# EUROPEAN PATENT APPLICATION

(11) **EP 4 682 237 A2**
(43) Date of publication of application: **21.01.2026**
(21) Application number: 25219900.5
(22) Date of filing: 14.04.2021
(51) Int. Cl.: C12M 1/00

(54) **SYSTEM FOR FUNCTIONAL TESTING AND TRANSPORTATION OF BIOLOGICALLY ENGINEERED ORGANS**

(30) Priority: 14.04.2020 US 202063009785 P
(62) Divisional of application: 21168393.3
(71) Applicant: Miromatrix Medical Inc., Eden Prairie, MN 55344 (US)
(72) Inventor: KATANE, Aleksandr, Minnesota 55318 (US)
(74) Representative: Murgitroyd & Company

(57) **Abstract**

A system (200) for providing external organ function to a patient in need, the system (200) comprising: a biologically engineered organ (50), a housing (204) configured to support the biologically engineered organ (50) therein in a perfusate flow, the housing including a perfusate inlet (204a) and a perfusate outlet (204b); a perfusate circuit (206) connected to the perfusate inlet (204a) and the perfusate outlet (204b) and configured to transmit perfusate through the system (200); and a pump (208) connected to thes perfusate circuit (206) and configured to circulate the perfusate through the perfusate circuit (206).

## Description

### BACKGROUND

Organ transplants are common across the United States and throughout the world. Organs such as livers, kidneys, pancreas, lungs and hearts are commonly transplanted to prolong the life of the recipients. However, there is often a shortage of organs, creating a demand and a waiting list for the organs. One solution to this shortage is to use biologically engineered organs and alternative tissues, where organs are stripped of their cells and the recipient's cells are perfused into the biologically engineered organ, to help prevent rejection.

### SUMMARY

The invention is a system for testing and transporting a biologically engineered organ and a method for testing and maintaining health of a biologically engineered organ during transportation of the biologically engineered organ as defined in the appended claims.

### BRIEF DESCRIPTION OF THE DRAWINGS

In the drawings, which are not necessarily drawn to scale, like numerals can describe similar components in different views. Like numerals having different letter suffixes can represent different instances of similar components. The drawings illustrate generally, by way of example, but not by way of limitation, various embodiments discussed in the present document.
FIG. 1 illustrates an overview of devices and systems involved in stages of transplanting a biologically engineered organ, in accordance with at least one example of the present disclosure.
FIG. 2 illustrates a schematic view of a system for functional testing and transportation of a bio-engineered organ, in accordance with at least one example of the present disclosure.
FIG. 3A illustrates a graph of a process monitored by a system, in accordance with at least one example of the present disclosure.
FIG. 3B illustrates a graph of a process monitored by a system, in accordance with at least one example of the present disclosure.
FIG. 3C illustrates a graph of a process monitored by a system, in accordance with at least one example of the present disclosure.
FIG. 3D illustrates a graph of a process monitored by a system, in accordance with at least one example of the present disclosure.
FIG. 3E illustrates a graph of a process monitored by a system, in accordance with at least one example of the present disclosure.
FIG. 4 illustrates a schematic view of a method, in accordance with at least one example of the present disclosure.
FIG.5 illustrates a block diagram of architecture for an example computing system used, according to at least one example of the present disclosure.

### DETAILED DESCRIPTION

Biologically engineered organs (BEOs) and advanced bio-engineered tissues (ATs) can help reduce rejection, helping to reduce organ wait times. BEOs can also help to reduce wait times by making use of organs of different species. In either scenario, the BEO is typically processed at a laboratory under tightly controlled conditions before it can be transported to a hospital for implantation. For example, ATs and BEOs should be conditioned with appropriate biochemical and physical cues to promote development and maintenance of physiological function throughout organ culture and transport to an operating suite. Functional release criteria testing should also be performed in order to ensure adequate function at the time of arrival in the operating suite to ensure appropriate in-vivo function and therapy.

As advanced tissues are bio-engineered and developed for therapy, there are a few challenges that concern both functional release qualification testing and the transportation of tissue to the site of implant for therapy. Advanced tissues (ATs) and bioengineered organs (BEOs) will need to demonstrate various levels of function prior to transportation to the site of implant. BEOs can include livers, lungs, hearts, pancreases, kidney, intestine, or the like. Additionally, these tissues need to have an advanced transport system that actively stimulates and monitors both the function and viability of the bio-engineered tissue en route to the destination. Finally, ATs and BEOs tend to be more delicate than native organs, so a transport system needs to provide greater protection against damage.

This disclosure discusses systems that can help address these issues by using a physical testing and transportation system that can accept an AT or BEO and can connect to the tissue's vascular system after the AT or BEO is matured in a bioreactor. The system can perfuse the AT or BEO with media or blood, when necessary. Once the AT or BEO has established active flow, the system can be used to test various function parameters. These functional tests can be specific to the AT or BEO that is installed into the system.

While the AT or BEO is being perfused, the system can be dosed with various chemicals, drugs, and/or proteins to challenge the AT or BEO for function. Samples of the media or blood can be collected and can be analyzed to determine functional adequacy. Once it is determined that the AT or BEO meets the functional requirements, the system can then continue to perfuse the AT or BEO as it is being transported to the site of implantation. The system can also actively monitor the housed AT or BEO for viability and function to ensure that it meets specifications prior to implantation. By using the same unit for both in-vitro testing and transport, the AT or BEO can remain in an uncompromising environment until the AT or BEO is removed from the system just prior to implantation.

The above discussion is intended to provide an overview of subject matter of the present patent application. It is not intended to provide an exclusive or exhaustive explanation of the invention. The description below is included to provide further information about the present patent application.

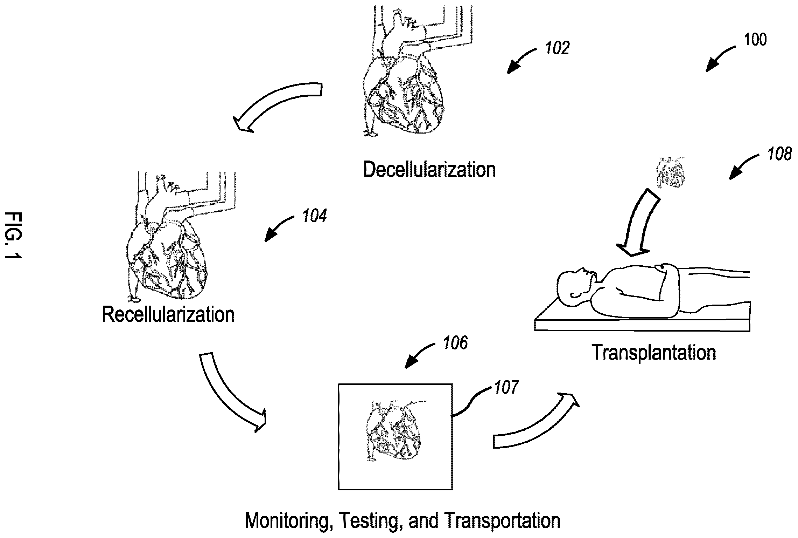

FIG. 1 illustrates an overview of devices and systems involved in stages of transplanting a BEO in accordance with at least one example of the present disclosure.

An example procedure 100 shows various stages that can be included in a transplantation procedure. At a stage 102, an organ can be received from a donor human or other species, such as pig, sheep, bovine, or the like. The organ can be decellularized in a laboratory under carefully controlled conditions, where all (or substantially all) cellular material can be removed from the organ. Following decellularization, the organ can be recellularized at a stage 104, whereby cells from the donor can be added to the organ and the organ can be functionally tested and otherwise prepared for implantation.

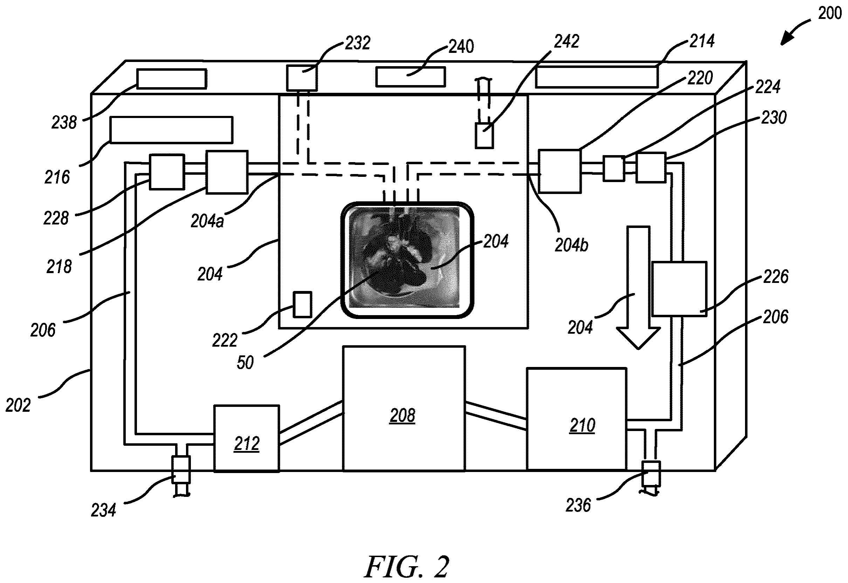

Once it is determined that the BEO is ready for transplant, the BEO can be prepared for transportation at stage 106. During this stage, the BEO can be secured within a transportation and testing system 107. During transportation, the BEO can be monitored and tested to ensure health of the BEO during transportation. Then, at step 108, the BEO can be received, such as by a surgical team who can receive data from the transport system confirming that the BEO is in condition for transplantation. The BEO can then be implanted into the recipient. Further details of several of these steps are discussed in further detail below with respect to FIGS. 2-4.

FIG. 2 illustrates a schematic view of a system 200 for functional testing and transportation of a bio-engineered organ (BEO), in accordance with at least one example of the present disclosure.

The system 200 can include an enclosure 202, a housing 204, a perfusate circuit 206, a pump 208, a heating/cooling system 210, a gas transfer system 212, a power source 214, a controller 216, an inlet temperature sensor 218, an outlet temperature sensor 220, a humidity sensor 222, a glucose sensor 224, an organ health sensor 226, an inlet CO2/O2 sensor 228, an outlet CO2/O2 sensor 230, a pressure sensor 232, a sampling port 234, a dosing port 236, a data connection 238, a user interface 240, and an ambient temperature sensor 242. Also shown in FIG. 2 is an organ 50, which can be a heart, for example.

The enclosure 202 can be a rigid or semi-rigid container, enclosure, or housing made of materials such as one or more of metals, plastics, foams, elastomers, ceramics, composites, combinations thereof, or the like. The enclosure 202 can include one or more latches, fasteners, or the like to resealably close. In some examples, the enclosure 202 can include one or more hinges. The enclosure 202 can include one or more insulative layers to help thermally isolate contents of the enclosure 202 from ambient conditions. The enclosure 202 can include a handle and/or a set of wheels such as for transportation of the enclosure 202 and its contents.

The housing 204 can be a rigid or semi-rigid body made of materials such as one or more of metals, plastics, foams, elastomers, ceramics, composites, combinations thereof, or the like. The housing 204 can be configured to receive and support a biologically engineered organ therein in a perfusate flow and/or bath. That is, the housing 204 can be shaped to support an organ therein. In some examples, the housing 204 can be organ-specific (such as kidney specific) such that the housing 204 is shaped to support the kidney (or other organ in other examples). In some examples, the organ-specific housing 204 can be removable and replaceable as required for an organ to be transported. The housing 204 can include a perfusate inlet 204a and a perfusate outlet 204b to receive perfusate flow through the housing 204. The enclosure 202 can be configured to surround and enclose at least a portion of the housing.

The perfusate circuit 206 can be connected to the perfusate inlet 204a and the perfusate outlet 204b and can be configured to transmit perfusate through the system and its components. The circuit 206 can be made of one or more types of tubing including rigid, semi-rigid, and flexible tubing on various materials (e.g., copper, steel, aluminum, plastics, silicone, or the like). Though perfusate is discussed generally with respect to FIG. 2, the perfusate circuit can be configured to handle media or blood. In some examples, patient-specific donor blood can be used in the circuit 206, which can help to qualify patient specific compatibility. In other examples, other medias can be used.

The pump 208 can be connected to the circuit 206 and can be configured to circulate perfusate through the perfusate circuit 206. The pump 208 can be a positive displacement pump, a centrifugal pump, or an axial pump. In some examples, the pump 208 can be a continuous type pump or a peristaltic type pump (with or without damping). In some examples, the pump 208 can be located downstream of the heating/cooling system 210 and upstream of the gas transfer unit 212.

The heating/cooling system 210 can be connected to the perfusate circuit 206 to exchange heat with the perfusate such that the perfusate is delivered to the housing within a target temperature range. The heating/cooling system 210 can be a heating system in some examples and can be a cooling system in some examples. In some examples, the system 210 can include discrete heating and cooling systems. The system 210 can be, for example, a refrigerant heat pump including a compressor, a condenser, an evaporator, one or more expansion valves, and a reversing valve. In some examples the system 210 can be a thermoelectric heating and cooling device (Peltier device). In some examples, the system can include a refrigeration cooling system and a resistive electric heating device. In any of these examples, the system 210 can include a heat exchanger configured to exchange heat with the perfusate indirectly.

The gas transfer unit 212 can be connected to the perfusate circuit 206 and configured to transfer gas to and from the perfusate, such as oxygen and carbon dioxide (CO2). The gas transfer unit 212 can be either an active gas transfer unit or a passive gas transfer unit configured to maintain oxygen and CO2 at a desired concentration range within the perfusate. In some examples, the gas transfer unit 212 can manage other gasses conducive to organ culturing. Optionally, the gas transfer unit 212 can include a gas storage unit, which can be a tank, vessel, or the like configured to store a gas or fluid. The gas storage unit of the gas transfer unit 212 can be configured to supply the gas transfer unit 212 with gas (such as oxygen) for transfer to the perfusate.

The power source 214 can be supported by the enclosure 202 and can be configured to power the pump 208, the heating/cooling system 210, the gas transfer unit 212, the various sensors of the system 200, the controller 216, the user interface 240, and/or other components of the system 200. In some examples, the power source 214 can be a replaceable battery, a rechargeable battery, or the like.

The inlet temperature sensor 218 can be connected to the perfusate circuit 206 upstream of the housing 204 (and can be within the housing 204) and upstream of the BEO. The outlet temperature sensor 220 can be connected to the perfusate circuit 206 downstream of the housing 204 (and can be within the housing 204) and downstream of the BEO. The ambient temperature sensor 242 can be connected to the enclosure 202 and positioned to measure a temperature ambient to the enclosure 200. Each of the inlet temperature sensor 218, the outlet temperature sensor 220, can be any type of fluid temperature sensor, either in a thermowell, coupled to a pipe of the circuit 206, or in direct contact with the process fluid, such as a thermistor, thermocouple, resistance temperature detector, or the like. The ambient temperature sensor 242 can be any type of air temperature sensor configured to measure an ambient dry bulb and/or wet bulb temperature.

The inlet temperature sensor 218 can be configured to produce an inlet temperature signal based on an inlet temperature of the perfusate entering the housing 204; the outlet temperature sensor 220 can be configured to produce an outlet temperature signal based on an outlet temperature of the perfusate leaving the housing 204; and, the ambient temperature sensor 242 can be configured to produce an ambient temperature signal based on an ambient temperature.

The humidity sensor 222 can be connected to the housing 204 and can be configured to produce a humidity signal based on a humidity internal to the housing. In some examples, the housing 204 can additionally or alternatively include a conductivity and/or level sensor configured to measure an amount of fluid within the housing 204. The humidity temperature sensor 222 can be configured to produce a humidity signal based on a humidity of the housing 204.

The glucose sensor 224 can be connected to the perfusate circuit 206, such as downstream of the housing 204. The glucose sensor 224 can be configured to produce a glucose sensor signal based on a glucose level of the perfusate. Similarly, the organ health sensor 226 can be connected to the perfusate circuit 206, such as downstream of the housing 204. The organ health sensor 226 can be configured to produce an organ health sensor signal based on a metabolite level of the perfusate within the circuit 206.

The inlet CO2/O2 sensor 228 can be connected to the perfusate circuit 206 upstream of the housing 204. The outlet CO2/O2 sensor 230 can be connected to the perfusate circuit 206 downstream of the housing 204. Each of the inlet CO2/O2 sensor 228 and the outlet CO2/O2 sensor can be a carbon dioxide sensor and an oxygen sensor configured to produce a carbon dioxide signal and/or an oxygen signal based on a respective CO2 level of the perfusate an O2 level of the perfusate.

The pressure sensor 232 can be connected to the perfusate circuit 206 downstream of the housing 204. The pressure sensor 232 can be configured to produce a pressure signal based on a pressure of the perfusate within the perfusate circuit 206.

The sampling port 234 can be connected to the perfusate circuit 206 (for example downstream of the pump 208) and can be configured to receive a syringe to withdraw a sample of the perfusate without compromising sterility of the perfusate within the circuit 206. Similarly, the dosing port 236 can be connected to the perfusate circuit 206 (for example upstream of the pump 208) and can be configured to receive supplements for delivery to the perfusate and ultimately the organ without compromising sterility of the perfusate within the circuit 206.

The data connection 238 can be any connection configured to transmit data therethrough such as between the controller 216, any of the sensors, and an external device. The connection can be, for example, ethernet, universal serial bus 2.0, firewire, or the like.

The controller 216 can be a programable controller, such as a single or multi-board computer, a direct digital controller (DDC), a programable logic controller (PLC), or the like. In other examples the controller 216 can be any computing device, such as a handheld computer, for example, a smart phone, a tablet, a laptop, a desktop computer, or any other computing device including a processor, memory, and communication capabilities.

The controller 216 can generally be configured to control operations of the systems 200, such as by controlling operation of the pump 208, the heating/cooling system 210, the gas transfer system 212, the power source 214, the controller 216, the inlet temperature sensor 218, the outlet temperature sensor 220, the humidity sensor 222, the glucose sensor 224, the organ health sensor 226, the inlet CO2/O2 sensor 228, the outlet CO2/O2 sensor 230, the pressure sensor 232, the sampling port 234, the dosing port 236, the data connection 238, and/or the user interface 240. Various examples of how the controller 216 can control the system 200 are discussed below.

The user interface 240 can be any display and/or input device. For example, user interface can be a touch screen display. In another example, user interface 240 can provide lights, buttons, and/or switches. The controller 216 and the user interface 240 can include machine readable medium. The terms "machine readable medium" can include any medium that is capable of storing, encoding, or carrying instructions for execution by the device and that cause the device to perform any one or more of the techniques of the present disclosure, or that is capable of storing, encoding or carrying data structures used by or associated with such instructions. Non-limiting machine readable medium examples can include solid-state memories, and optical and magnetic media. Specific examples of machine readable media can include: non-volatile memory, such as semiconductor memory devices (e.g., Electrically Programmable Read-Only Memory (EPROM), Electrically Erasable Programmable Read-Only Memory (EEPROM)) and flash memory devices; magnetic disks, such as internal hard disks and removable disks; magneto-optical disks; and CD-ROM and DVD-ROM disks.

In one example, the controller 216 can be configured to receive the inlet temperature signal from the inlet temperature sensor 218 and the outlet temperature signal from the outlet temperature sensor 220. The controller 216 can further be configured to operate the pump 208, the gas transfer unit 212, and the heating and cooling system 210 based on the inlet temperature signal and the outlet temperature signal. For example, the controller 216 can activate the cooling system 210 when the outlet temperature signal indicates that a temperature of the perfusate in the circuit 206 is above a threshold, for example 37, 38, or 39 degrees Celsius. Similarly, the controller 216 can activate the heating system 210 when the outlet temperature signal indicates that a temperature of the perfusate in the circuit 206 is below a threshold, for example 37, 36, or 35 degrees Celsius. In some examples, the controller 216 can modify operation of such components based on the ambient temperature signal from the ambient temperature sensor 242. The controller 216 can also be configured to produce an alert based on any of the temperature sensor signals.

In another example, the controller 216 can be configured to receive the humidity signal from the humidity sensor 222 and the controller 216 can be configured to operate the pump 208, the gas transfer unit 212, and the heating and cooling system 210 based on the humidity signal. It can be desired to keep the housing 204 at an environment with a humidity between 80% and 100% relative humidity and relative humidities below this range can be adverse to the health of the organ. For example, if a humidity of the housing 204 drops below a threshold (for example 75% relative humidity), the controller 216 can activate the pump 208 to circulate perfusate through the housing 204. The controller 216 can also be configured to produce an alert based on the humidity sensor signal.

In another example, the controller 216 can be configured to receive the organ health sensor signal from the organ health sensor 226 and the controller 216 can be configured to operate the pump 208 and the gas transfer unit 212 based on the organ health sensor signal. The controller 216 can also be configured to produce an alert based on the organ health sensor signal. In some examples, the organ health sensors can me a metabolite sensor or sensors.

The organ health sensor 226 can be various types of sensors configured to monitor one or more conditions of the perfusate. For example, the organ health sensor 226 can be an ammonia sensor configured to produce a signal based on an ammonia concentration or level of the perfusate. Also, the organ health sensor 226 can be a glutamine sensor configured to produce a signal based on a glutamine concentration or level of the perfusate. Also, the organ health sensor 226 can be a lactate sensor configured to produce a signal based on a lactate concentration or level of the perfusate. Also, the organ health sensor 226 can be a pH sensor configured to produce a signal based on a pH of the perfusate. Also, the organ health sensor 226 can be a bile sensor configured to produce a signal based on a bile concentration or level of the perfusate. Also, the organ health sensor 226 can be a creatinine sensor configured to produce a signal based on a creatinine concentration or level of the perfusate. Also, the organ health sensor 226 can be an albumin sensor configured to produce a signal based on an albumin concentration or level of the perfusate.

Also, the organ health sensor 226 can be a coagulation time sensor to produce a signal based on an active coagulation time (ACT) of the blood. Clotting factors, such as (Factor VII, Factor VIII, or Factor X), can also be monitored in blood, perfusate, or media. In some examples, the controller 216 can be configured to receive the health signal from the health sensor and can be configured to control an injection of an anticoagulant (such as heparin) if the ACT falls below a threshold (such as below 100 seconds).

In another example, the controller 216 can be configured to receive the glucose sensor signal from the glucose sensor 224 and the controller 216 can be configured to operate the pump 208 and the gas transfer unit 212 based on the glucose sensor signal. For example, if a glucose concentration of the perfusate in the circuit 206 drops below a threshold (for example 0.5 grams per liter), the controller 216 can activate a glucose injection pump to inject glucose into the dosing port 236 and into the perfusate circuit 206. The controller 216 can also be configured to produce an alert based on the glucose sensor signal, such as if a glucose consumption rate of the organ 50 drops below a threshold (for example 100 milligrams per hour).

In another example, the controller 216 can be configured to receive the pressure signal from the pressure sensor 232 and the controller 216 can be configured to operate the pump 208 based on the pressure signal. The controller 216 can also be configured to produce an alert based on the pressure signal, such as if the pressure drops below a threshold pressure and the pump is operating, indicating that the pump is failing or has failed.

In another example, the controller 216 can be configured to receive the inlet temperature signal from the inlet temperature sensor 218 and/or receive the outlet temperature signal from the outlet temperature sensor. And, the controller 216 can be configured to operate the pump 208, the gas transfer unit 212, and the heating and cooling system 210 based on the inlet temperature signal and the outlet temperature signal.

In operation of some examples, the system 200 can be configured to combine functional testing and transport of a BEO. Once the AT or BEO is connected to the system, media or blood can be introduced into the AT or BEO and can be perfused through connected vasculature of the BEO. A flow rate of the perfusate and the pressure of the perfusate can be monitored by the system 200 (using the pressure sensor signal from the pressure sensor 232) and the pump 208 can be adjusted to control the flow rate and the pressure. The flow rate, pressure, and temperature of the perfusate can be monitored at various positions in the perfusate circuit 206. Though the sensors are shown in FIG. 2 as having specific locations, the sensors can be positioned in various locations in other examples.

The flow rate and pressure of the perfusate are examples of physical cues that can promote development and maintenance of function of the AT or the BEO (the organ 50). These dosing port 236 can be used to introduce various substances to the perfusion circuit 206. For example, the dosing port 236 can receive glucose if the controller 216 determines, based on the glucose signal, that a glucose level within the perfusate is below a threshold. In some examples, the dosing port 236 can be connected to one or more linear pump systems that are able to inject a specific volume dose over a specific period of time. Similarly, the sampling port 234 can be connected to one or more linear pump system that are able to sample a specific volume over a specific period of time.

In some examples, the sampling port 234 can be used to take a sample volume out of the perfusate circuit. The sample can be manually tested and the sample can be automatically tested for sampling and analysis during transport. Samples collected from the sampling port 234 can be used by the controller 216 to add biochemical components through the dosing port 236 that are necessary to develop and maintain the function of the AT or BEO. Further, automatic sampling of sub-systems can be performed by the controller. For example, blood can be automatically drawn from the system and can be delivered to a component that measures ACT, glucose, ammonia, or the like.

The system 200 can also include multiple subsystems, as described above, to test and maintain the media or blood. For example, the heating/cooling system 210 can be operated by the controller 216 to keep the media or blood warm (or cool) during transport. The gas transfer module 210 can help maintain the CO2 and/or O2 in the perfusate or blood.

Ex-vivo functionality tests can be performed inside the system 200 that meet adequate in-vivo function specifications specific to the AT or BEO. For example, once a liver or kidney BEO is installed (as the organ 50), pressure at defined flow rates can be monitored (such as by using the pressure signal from the pressure sensor 232) as a sign of patency over time. If the pressure of the blood or perfusate perfused through the system 200 at a specific flow rate does not exceed a specified threshold pressure over a specified period of test time, the liver or kidney BEO can be designated as patent by the controller 216, which can communicate as much by displaying that the organ 50 is patent on the user interface 240 or can transmit a message externally informing as much. In addition, pressure and perfusion rates during transportation can provide final release criteria to a surgery team prior to transplantation of the organ 50, such as through the user interface 240 or an external device.

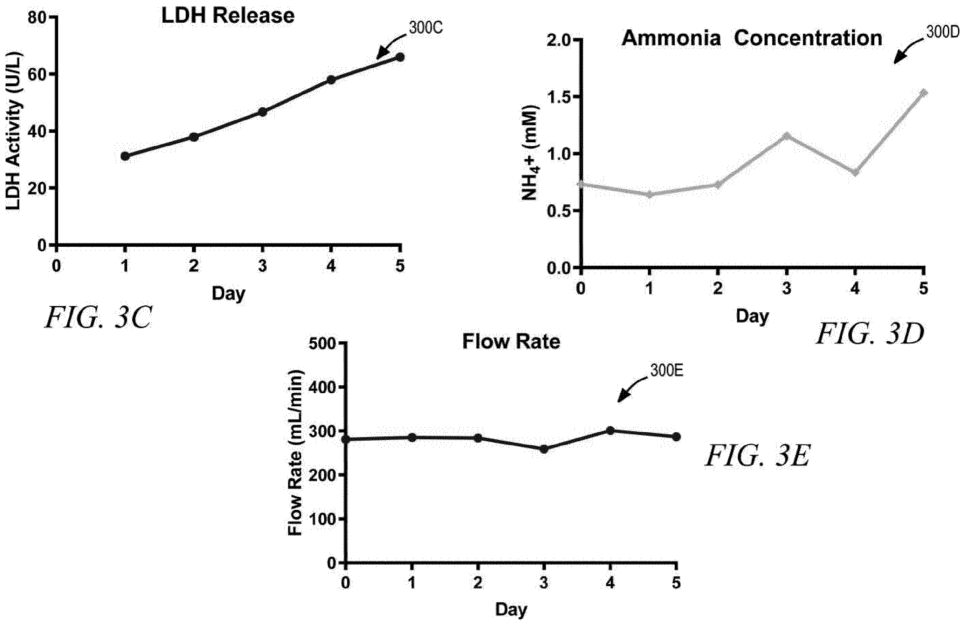

One important function of liver BEOs is to clear ammonia. Once a liver BEO is installed within the system 200, the perfusate of the circuit 206 can be sampled periodically (such as using the metabolite sensor 226) and analyzed by the controller 216 for ammonia levels to ensure the ammonia levels are within a desired range, as shown in FIG. 3D. Depending on the ammonia level, a specified dose of ammonium chloride (NH₄Cl), ammonium sulfate, or similar ammonia substrate, can be injected over a specified period of time through the dosage port 236. Additionally, or alternatively, samples of the blood or media can be taken periodically (such as through the sampling port 234) and can be bioanalyzed to determine if the liver BEO (the organ 50) is clearing ammonia at an adequate rate that meets release criteria. Additionally, or alternatively, samples of the blood or media can be taken and assayed for urea content over a specified period of time in order to determine if the liver BEO (the organ 50) has adequate urea production that meets release specification criteria.

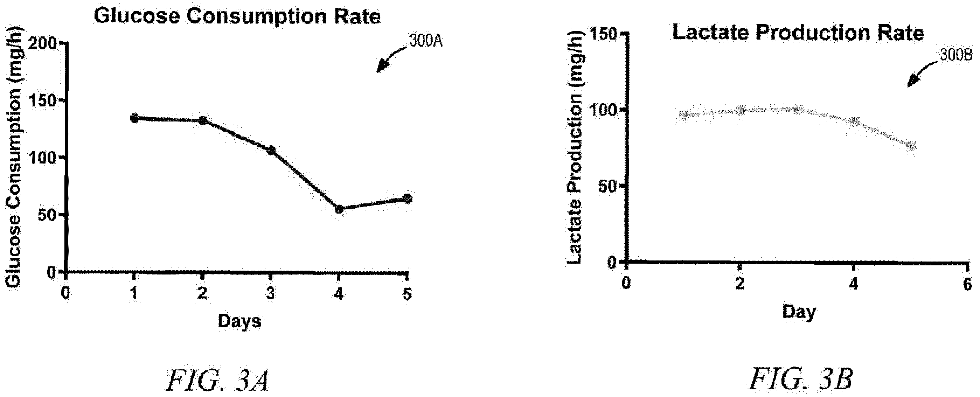

In some examples, samples of the blood or media can be taken through the sampling port 234 (or using the glucose sensor 224). The samples can be assayed (such as by the controller 216) for glucose content of the perfusate over a specified period of time, as shown in FIG. 3A. This can be done in order to determine if the AT or BEO has the adequate glucose consumption rate that meets release specification criteria. When it is determined that the glucose level of the perfusate in the circuit 206 is too low, the controller 216 can operate an in-line injector to inject glucose into the circuit 206 through the injection port 236.

In some examples, a specified dose of drugs commonly cleared in the liver can be injected over a specified period of time ((R)-Warfarin, (S)-Mephenytoin, Acetaminophen, Aprepitant, Azole, Benzodiazepines, Beta, Caffeine, Calcium, Carbamazepine, Celecoxib, Clarithromycin, Codeine, Cyclosporine, Delavirdine, Dextromethorphan, Diazepam, Diclofenac, Enalapril, Erythromycin, Estradiol, Estrogen, Fentanyl, Finasteride, Flecainide, Fluoxetine, Glipizide, Glyburide, Haloperidol, Indinavir, Indomethacin, Lidocaine, Lopinavir, Loratidine, Methadone, Mexiletine, Morphine, Nelfinavir, Nifedipine, Olanzapine, Omeprazole, Opioid, Pentamidine, Phenothiazines, Phenytoin, Piroxicam, Prednisone, Progesterone, Propranolol, Quinidine, Risperidone, Ritonavir, Selective serotonin reuptake inhibitors, Saquinavir, Sildenafil, Sirolimus, Statins, Tacrine, Tacrolimus, Tamoxifen, Testosterone, Theophylline, Tramadol, Trazodone, Tricyclic, Valproate, Venlafaxine, Verapamil, or Voriconazole.). Samples of the blood or media can be taken from the sampling port 234 periodically and can be bioanalyzed to determine if the liver BEO (the organ 50) is clearing the drugs to at an adequate rate that meets release criteria. In some examples, samples of the blood or media can be taken from the sampling port 234 and assayed for albumin content over a specified period of time in order to determine if the liver BEO has adequate albumin production that meets release specification criteria. In some examples, samples of the blood or media can be taken from the sampling port 234 and assayed for albumin content over a specified period of time in order to determine if the liver BEO has adequate bile production that meets release specification criteria. In some examples, samples of the blood or media can be taken from the sampling port 234 and can be assayed for creatinine, BSA, and/or urea content over a specified period of time in order to determine if the kidney BEO (the organ 50) has the glomerular filtration rate that meets release specification criteria.

Once it is determined that the matured AT or BEO (the organ 50) meets release criteria, the system 200 can then be used as an active transport vessel. Media or blood can be actively perfused through the AT or BEO (the organ 50) as the system 200 is transported to the site of implant. During transportation, the system 50 can sample the blood or media (using the sampling port 234 and/or the sensors of the system 200) in order to continuously monitor viability and function of the organ 50. The blood or media samples can be analyzed for glucose, glutamine, ammonia, LDH, albumin, urea and/or creatinine. In some examples, the system 200 can use the organ health sensor 226 to measure ammonia, glutamine, pH, bile, creatinine, albumin, urea, LDH, oxygen, or the like. Values can be logged by the controller 216 and can be monitored for minimums, maximum, trends, or the like. When a datum or data of the monitored criteria are outside of a designated range, an alert can be produced by the controller 216 and can be transmitted to an external device. Similarly, the controller can display the alert on the display (such as the display 510 discussed below).

Further, the perfusion flow rate can be regulated by the controller 216 using the pump 208, and the pressure can also be monitored (through the pressure sensor 232) throughout the transport process in order to analyze AT or BEO patency. During transport, the system 200 can be dosed with nutrients and other transport maintenance formulations, through the dosing port 236, in order to sustain the AT or BEO (the organ 50) for the duration of time prior to implantation.

The housing 204 for the AT or BEO (the organ 50) inside of the enclosure 202 can allow for sterile installation of the AT or BEO. The housing 204 can also allow for the sterile removal of the AT or BEO (the organ 50) for implantation. Components of the system 200 can enable the organ 50 to remain at a physiological temperature during transportation by measuring the inlet temperature of the perfusate using the inlet temperature sensor 218 and the outlet temperature sensor 220. The controller 216 can use signals from these sensors to control the heating/cooling system 210 to maintain a temperature of the organ 50 in a physiological range (e.g., 37 degrees Celsius, ± 1 degree). The moisture level of the organ can also be measured using the humidity sensor 222 and can be regulated by the controller 216 using the pump 208.

In some examples, the system 200 can add challenge substrates, such as automatically through a pump and the injection port 236 (as controlled by the controller 216) or manually through the injection port 236. The challenge substrates can be used to measure function of the BEO during transport or prior to implantation. In one example, a pump connected to the injection port 236 can add a bolus of ammonia to the circuit 206 for circulation to the BEO (organ 50). Then, the organ health sensor 226, which can be an ammonia sensor configured to produce a signal based on an ammonia concentration or level of the perfusate, can detect an ammonia concentration of the perfusate and transmit the organ health signal to the controller 216. The controller can log and analyze the organ health signal to determine a clearance rate of ammonia by the BEO, such as a percentage change within a defined time period. The controller can use the data and clearance rates as an in-process for functional release of the BEO. In other examples, a similar process can be used for other substrates for Liver, Kidney, Lung, pancreas, intestine, heart, or any organ. The substrates can include, glucose, glutamine, various metabolites, ammonia acids, vasodilators, oxygen, ammonia, creatine, urea, albumin, oxygen, hormones, cytokines, or the like. FIG. 3A illustrates a graph 300A of a process monitored by a system, such as the system 200, in accordance with at least one example of the present disclosure. The graph 300A shows glucose consumption of the organ 50 within the system 200, where the glucose levels can be measured by the glucose sensor 224. The controller 216 can monitor the glucose levels over time and can adjust operation of the system 200 based on the glucose levels.

Optionally, the system 200 can be configured to connect to and support an organ located external to the enclosure 202 through patient access lines that can extend beyond the enclosure 202. For example, the system 200 can connect to a liver of a patient. The liver can be connected to the system 200 via the perfusate inlet 204a and the perfusate outlet 204b, which can optionally extend through the enclosure 202. Optionally, the system 200 can include an external discharge system 244 and an external inlet system 246. The discharge system 244 can include a discharge line 248, a valve 250, and a pressure sensor 252. The inlet system 246 can include an inlet line 254, a valve 256, and a storage tank 256. Though the discharge line 248 is shown as being connected to a downstream portion (e.g., discharge) of the pump 208 and the inlet line is shown as being connected to an upstream side (e.g., inlet) of the pump 208, the discharge line 248 and the inlet line 254 can be connected to various locations in the system 200.

The lines 248 and 254 can be made of one or more types of tubing including rigid, semi-rigid, and flexible tubing on various materials (e.g., copper, steel, aluminum, plastics, silicone, or the like). The valves 250 and 256 can be isolation valves or control valves such as ball valves, gate valves, butterfly valves, or the like. Optionally, the valves 250 and 256 can include an actuator in communication with the controller 216 such that the controller 216 can modulate flow rates or pressures of perfusate through the lines 248 and 254 using the valves 250 and 256, respectively. Optionally, the valves 250 and 256 can be located within the enclosure 202.

The pressure sensor 252 can be connected to the outlet line 248. The pressure sensor 252 can be configured to produce a pressure signal based on a pressure of the perfusate within the outlet system 244. The inlet system 246 can optionally include a pressure sensor configured to produce a pressure signal based on a pressure of the perfusate within the inlet system 246.

The tank 258 can be connected to the inlet system 246 and can be configured to store fluid (e.g., blood, perfusate, or other fluids) therein. The tank 258 can be a storage tank configured to store additional fluid to help fill the lines 248 and 254 and any other lines connected thereto between the organ and the valves 250 and 256. The tank 258 can also be an expansion tank, which can include a bladder or the like, to help adjust a pressure of the system 200, such as based on an operating volume of the system.

In some examples, the pump 208 can include multiple pumps, such as a primary pump and secondary pump arrangement. Alternatively, the pump 208 can include a primary, secondary, tertiary pump arrangement. In one such example of a primary and secondary system, a main pump 208a can operate to circulate perfusate through the circuit 206 (such as in the enclosure) to allow the maintenance and monitoring functions to be performed by the controller 216. In this example, a secondary pump 208b can be included and connected to the inlet system 246 and the outlet system 244 to circulate fluid therebetween and to an external organ. A bypass line 260 can optionally be included for the main pump to bypass the inlet system 244 and the outlet system. The bypass line 260 can optionally include a control valve

In operation of such an example, an organ (e.g., a liver) can be connected to the inlet system 246 and the outlet system 244 to allow the system 200 to perform maintenance or monitoring of the external organ using the system 200. For example, the controller 216 can be used to monitor the organ health using one or more of the various sensors, such as the inlet temperature sensor 218, the outlet temperature sensor 220, the humidity sensor 222, the glucose sensor 224, the organ health sensor 226, the inlet CO2/O2 sensor 228, the outlet CO2/O2 sensor 230, the pressure sensor 232, the ambient temperature sensor 242, the pressure sensor 252, or the like.

Optionally, when the external organ is connected to the lines 248 and 254, the sampling port 234 can be used to collect a sample of the perfusate such as for analysis performed externally to the system 200. Optionally, the dosing port 236 can be used for delivery of one or more solutions to the perfusate, such as to introduce various substances to the perfusion circuit 206. For example, the dosing port 236 can receive glucose if the controller 216 determines, based on the glucose signal, that a glucose level within the perfusate is below a threshold.

In operation of some example, the controller 216 can use the pressure sensor to determine a pressure in the discharge line 248 or the inlet line 254. The controller can control operation of the pump (e.g., pump speed) to adjust one or more of the inlet or discharge pressure. Such pressure control can help to limit damage caused to the system (e.g., a human body) currently supporting the organ that is external to the housing 204.

In operation, the external organ can be flushed of transport solution using the system 200 (e.g., the pumps 208), and then can be primed with compatible blood to the recipient or directly with the patient's blood. The external organ can remain connected to the patient for 1, 2, 3, 4, 5, 6, 7, 8, 9, 10, 11, 12,13, 14 or more days. The external organ can remain connected for a range of 2-7 days. During this time, the external organ can be monitored and the controller 216 can be used to monitor the organ and make the appropriate pressure, flow, oxygen and or nutrient corrections.

The external organ can help to provide temporary liver function such as reduced ammonia levels, increased albumin, drug detoxification, increased clotting factors, glucose stability, and any other important liver function. This could be utilized for acute liver failure patients.

The external organ can help to provide temporary kidney function similar to dialysis such as for creatine clearance, albumin retention, water reduction, blood pressure maintenance, EPO production, or any other kidney function. This could be utilized for chronic kidney failure patients.

In such an example, the external organ can be connected to the system 200 to precondition the organ prior to transplant. During this period the organ can be monitored for pressure, flow, vascular resistance, rejection, bleeding, defects, metabolic function, organ function such as ammonia clearance, bile production, urine production, heart output, lung oxygenation, or any organ specific function. Following 1, 2, 3, 4, 5, 6, 7, 8, 9, 10, 11, 12,13, 14 or more days (with an optimal range of 2-7 days) the organ can be removed from the housing and surgically transplanted into the patient. The preconditioning step can allow the organ function to continuously monitored, precondition, allow for functional improvement if needed, prior to being implanted.

Any of the operations of the controller 216 discussed above or below can be incorporated into control, maintenance, or monitoring of an organ external to the housing 204.

FIG. 3B illustrates a graph 300B of a process monitored by a system, such as the system 200, in accordance with at least one example of the present disclosure. The graph 300B shows lactate production of the organ 50 within the system 200, where the lactate levels can be measured through the sampling port 234 and/or through the metabolite sensor 226. The controller 216 can monitor the lactate levels over time and can adjust operation of the system 200 based on the lactate levels.

FIG. 3C illustrates a graph 300C of a process monitored by a system, such as the system 200, in accordance with at least one example of the present disclosure. The graph 300C shows lactate dehydrogenase (LDH) release of the organ 50 within the system 200, where the LDH levels can be measured through the sampling port 234 and/or through the metabolite sensor 226. The controller 216 can monitor the LDH levels over time and can adjust operation of the system 200 based on the LDH levels.

FIG. 3D illustrates a graph 300D of a process monitored by a system, such as the system 200, in accordance with at least one example of the present disclosure. The graph 300D shows ammonia concentration of the perfusate within the circuit 206, where the ammonia concentration can be measured by through the sampling port 234 and/or through the metabolite sensor 226. The controller 216 can monitor the ammonia concentration levels over time and can adjust operation of the system 200 based on the ammonia concentration levels.

FIG. 3E illustrates a graph 300E of a process monitored by a system, in accordance with at least one example of the present disclosure. The graph 300E shows flow rate of perfusate to the organ 50 within the system 200, where the flow rate can be measured by through the pressure sensor 232 and/or through operation of the pump 208 (using a flow curve of the pump). In some examples, the pressure sensor 232 can be a flow meter (such as a paddle-type, Coriolis type, or the like), and can be configured to directly measure a flow rate of the perfusate through the circuit 206. In either example, the controller 216 can monitor the flow rate of the perfusate through the circuit 206 over time and can adjust operation of the pump 208 (and other components of the system 200) based on the flow rate.

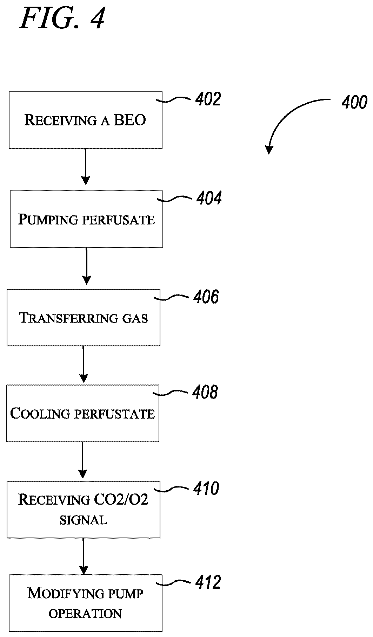

FIG. 4 illustrates a schematic view of a method 400, in accordance with at least one example of the present disclosure. The method 400 can be a method of testing and maintaining health of a BEO during transportation of the BEO. The steps or operations of the method 400 are illustrated in a particular order for convenience and clarity; many of the discussed operations can be performed in a different sequence or in parallel without materially impacting other operations. The method 1500 as discussed includes operations performed by multiple different actors, devices, and/or systems. It is understood that subsets of the operations discussed in the method 400 can be attributable to a single actor, device, or system could be considered a separate standalone process or method.

Method 400 can begin at step 402, wherein a BEO can be received and supported in a housing. For example, the organ 50 can be received and supported by the housing 204. At step 404, the perfusate can be pumped (using a pump) through a perfusate circuit connected to the housing to flow perfusate through the housing. For example, perfusate can be pumped by the pump 208 through the perfusate circuit 206 and therefore to the housing 204 to flow perfusate through the housing 204.

At step 406, gas can be transferred to and/or from the perfusate using a gas transfer unit connected to the perfusate circuit. For example, gas can be transferred to and/or from the perfusate using the gas transfer unit 212 connected to the perfusate circuit 216. At step 408, the perfusate can be cooled using a cooling system connected to the perfusate circuit. For example, the perfusate can be cooled using the cooling system 210 connected to the perfusate circuit 206.

At step 410 a carbon dioxide signal and an oxygen signal can be received from a carbon dioxide and oxygen sensor connected to the perfusate circuit. For example, a carbon dioxide signal and/or an oxygen signal can be received from the carbon dioxide sensor and the oxygen sensor (228 and/or 230) connected to the perfusate circuit 206. At step 412, operation of the pump can be modified and/or operation of the gas transfer unit can be modified based on the carbon dioxide signal and the oxygen signal. For example, operation of the pump 208 can be modified and/or operation of the gas transfer 212 unit can be modified based on the carbon dioxide signal and the oxygen signal.

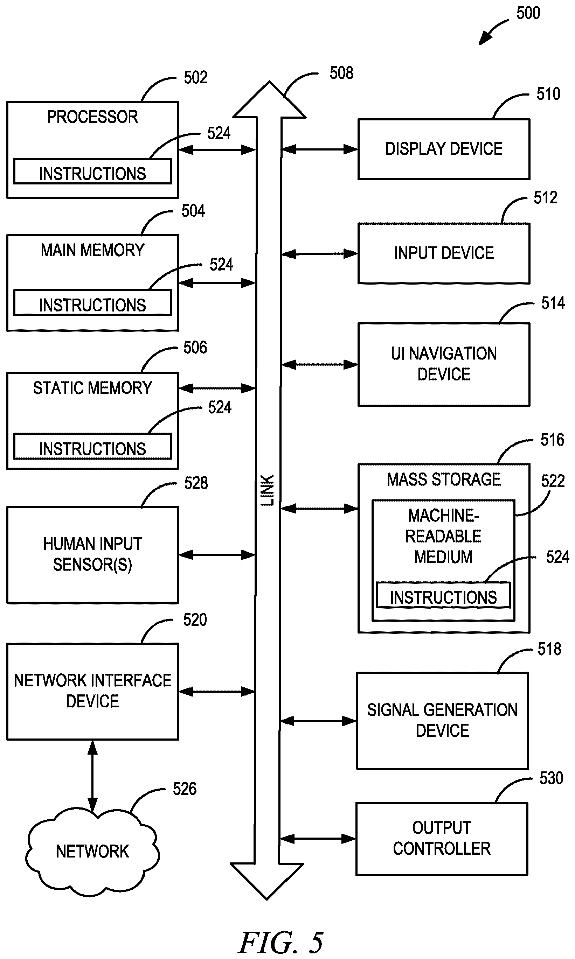

FIG. 5 illustrates a block diagram of an example machine 500 upon which any one or more of the techniques (e.g., methodologies) discussed herein can perform. Examples, as described herein, can include, or can operate by, logic or a number of components, or mechanisms in the machine 500 (which can be the system 200). Circuitry (e.g., processing circuitry) is a collection of circuits implemented in tangible entities of the machine 500 that include hardware (e.g., simple circuits, gates, logic, etc.). Circuitry membership can be flexible over time. Circuitries include members that may, alone or in combination, perform specified operations when operating. In an example, hardware of the circuitry can be immutably designed to carry out a specific operation (e.g., hardwired). In an example, the hardware of the circuitry can include variably connected physical components (e.g., execution units, transistors, simple circuits, etc.) including a machine readable medium physically modified (e.g., magnetically, electrically, moveable placement of invariant massed particles, etc.) to encode instructions of the specific operation. In connecting the physical components, the underlying electrical properties of a hardware constituent are changed, for example, from an insulator to a conductor or vice versa. The instructions enable embedded hardware (e.g., the execution units or a loading mechanism) to create members of the circuitry in hardware via the variable connections to carry out portions of the specific operation when in operation. Accordingly, in an example, the machine readable medium elements are part of the circuitry or are communicatively coupled to the other components of the circuitry when the device is operating. In an example, any of the physical components can be used in more than one member of more than one circuitry. For example, under operation, execution units can be used in a first circuit of a first circuitry at one point in time and reused by a second circuit in the first circuitry, or by a third circuit in a second circuitry at a different time. Additional examples of these components with respect to the machine 500 follow.

In alternative embodiments, the machine 500 can operate as a standalone device or can be connected (e.g., networked) to other machines. In a networked deployment, the machine 500 can operate in the capacity of a server machine, a client machine, or both in server-client network environments. In an example, the machine 500 can act as a peer machine in peer-to-peer (P2P) (or other distributed) network environment. The machine 500 can be a personal computer (PC), a tablet PC, a set-top box (STB), a personal digital assistant (PDA), a mobile telephone, a web appliance, a network router, switch or bridge, or any machine capable of executing instructions (sequential or otherwise) that specify actions to be taken by that machine. Further, while only a single machine is illustrated, the term "machine" shall also be taken to include any collection of machines that individually or jointly execute a set (or multiple sets) of instructions to perform any one or more of the methodologies discussed herein, such as cloud computing, software as a service (SaaS), other computer cluster configurations.

The machine (e.g., computer system) 500 can include a hardware processor 502 (e.g., a central processing unit (CPU), a graphics processing unit (GPU), a hardware processor core, or any combination thereof), a main memory 504, a static memory (e.g., memory or storage for firmware, microcode, a basic-input-output (BIOS), unified extensible firmware interface (UEFI), etc.) 506, and mass storage 508 (e.g., hard drive, tape drive, flash storage, or other block devices) some or all of which can communicate with each other via an interlink (e.g., bus) 530. The machine 500 can further include a display unit 510, an alphanumeric input device 512 (e.g., a keyboard), and a user interface (UI) navigation device 514 (e.g., a mouse). In an example, the display unit 510, input device 512 and UI navigation device 514 can be a touch screen display. The machine 500 can additionally include a storage device (e.g., drive unit) 508, a signal generation device 518 (e.g., a speaker), a network interface device 520, and one or more sensors 516, such as a global positioning system (GPS) sensor, compass, accelerometer, or other sensor. The machine 500 can include an output controller 528, such as a serial (e.g., universal serial bus (USB), parallel, or other wired or wireless (e.g., infrared (IR), near field communication (NFC), etc.) connection to communicate or control one or more peripheral devices (e.g., a printer, card reader, etc.).

Registers of the processor 502, the main memory 504, the static memory 506, or the mass storage 508 can be, or include, a machine readable medium 522 on which is stored one or more sets of data structures or instructions 524 (e.g., software) embodying or utilized by any one or more of the techniques or functions described herein. The instructions 524 can also reside, completely or at least partially, within any of registers of the processor 502, the main memory 504, the static memory 506, or the mass storage 508 during execution thereof by the machine 500. In an example, one or any combination of the hardware processor 502, the main memory 504, the static memory 506, or the mass storage 508 can constitute the machine readable media 522. While the machine readable medium 522 is illustrated as a single medium, the term "machine readable medium" can include a single medium or multiple media (e.g., a centralized or distributed database, and/or associated caches and servers) configured to store the one or more instructions 524.

The term "machine readable medium" can include any medium that is capable of storing, encoding, or carrying instructions for execution by the machine 500 and that cause the machine 500 to perform any one or more of the techniques of the present disclosure, or that is capable of storing, encoding or carrying data structures used by or associated with such instructions. Non-limiting machine readable medium examples can include solid-state memories, optical media, magnetic media, and signals (e.g., radio frequency signals, other photon based signals, sound signals, etc.). In an example, a non-transitory machine readable medium comprises a machine readable medium with a plurality of particles having invariant (e.g., rest) mass, and thus are compositions of matter. Accordingly, non-transitory machine-readable media are machine readable media that do not include transitory propagating signals. Specific examples of non-transitory machine readable media can include: non-volatile memory, such as semiconductor memory devices (e.g., Electrically Programmable Read-Only Memory (EPROM), Electrically Erasable Programmable Read-Only Memory (EEPROM)) and flash memory devices; magnetic disks, such as internal hard disks and removable disks; magneto-optical disks; and CD-ROM and DVD-ROM disks.

The instructions 524 can be further transmitted or received over a communications network 526 using a transmission medium via the network interface device 520 utilizing any one of a number of transfer protocols (e.g., frame relay, internet protocol (IP), transmission control protocol (TCP), user datagram protocol (UDP), hypertext transfer protocol (HTTP), etc.). Example communication networks can include a local area network (LAN), a wide area network (WAN), a packet data network (e.g., the Internet), mobile telephone networks (e.g., cellular networks), Plain Old Telephone (POTS) networks, and wireless data networks (e.g., Institute of Electrical and Electronics Engineers (IEEE) 802.11 family of standards known as Wi-Fi^{®}, IEEE 802.16 family of standards known as WiMax^{®}), IEEE 802.15.4 family of standards, peer-to-peer (P2P) networks, among others. In an example, the network interface device 520 can include one or more physical jacks (e.g., Ethernet, coaxial, or phone jacks) or one or more antennas to connect to the communications network 526. In an example, the network interface device 520 can include a plurality of antennas to wirelessly communicate using at least one of single-input multiple-output (SIMO), multiple-input multiple-output (MIMO), or multiple-input single-output (MISO) techniques. The term "transmission medium" shall be taken to include any intangible medium that is capable of storing, encoding or carrying instructions for execution by the machine 500, and includes digital or analog communications signals or other intangible medium to facilitate communication of such software. A transmission medium is a machine readable medium.

### NOTES AND EXAMPLES

The following, non-limiting examples, detail certain aspects of the present subject matter to solve the challenges and provide the benefits discussed herein, among others.

Example 1 is a system for testing and transporting a biologically engineered organ, the system comprising: a housing configured to receive and support a biologically engineered organ therein in a perfusate flow, the housing including a perfusate inlet and a perfusate outlet to receive the perfusate flow through the housing; a transportable enclosure surrounding at least a portion of the housing, the enclosure configured to insulate the housing; a perfusate circuit connected to the perfusate inlet and the perfusate outlet and configured to transmit perfusate through the system; a pump connected to the circuit and configured to circulate perfusate through the perfusate circuit; a gas transfer unit connected to the perfusate circuit and configured to transfer gas to and from the perfusate; a glucose sensor connected to the circuit downstream of the housing, the glucose sensor configured to produce a glucose sensor signal based on a glucose level of the perfusate; and a controller configured to operate the pump and the gas transfer unit based on the glucose signal.

In Example 2, the subject matter of Example 1 includes, a heating and cooling system connected to the perfusate circuit to exchange heat with the perfusate such that the perfusate is delivered to the housing within a target temperature range.

In Example 3, the subject matter of Example 2 includes, an inlet temperature sensor connected to the circuit upstream of the housing, the inlet temperature sensor configured to produce an inlet temperature signal based on an inlet temperature of the perfusate entering the housing; and an outlet temperature sensor connected to the circuit downstream of the housing, the outlet temperature sensor configured to produce an outlet temperature signal based on an outlet temperature of the perfusate leaving the housing.

In Example 4, the subject matter of Example 3 includes, a controller configured to: receive the inlet temperature signal and the outlet temperature signal; and operate the pump, the gas transfer unit, and the heating and cooling system based on the inlet temperature signal and the outlet temperature signal.

In Example 5, the subject matter of Examples 2-4 includes, a humidity sensor connected to the housing, the humidity sensor configured to produce a humidity signal based on a humidity of the housing; and a controller configured to receive the humidity signal and operate the pump, the gas transfer unit, and the heating and cooling system based on the humidity signal.

In Example 6, the subject matter of Examples 1-5 includes, a power source supported by the enclosure and configured to power the pump and the gas transfer unit.

In Example 7, the subject matter of Examples 3-6 includes, a metabolite sensor connected to the circuit downstream of the housing, the metabolite sensor configured to produce a metabolite sensor signal based on a metabolite level of the perfusate.

In Example 8, the subject matter of Examples 4-7 includes, a controller configured to: receive the metabolite sensor signal and the glucose sensor signal; and operate the pump and the gas transfer unit based on the metabolite sensor signal and the glucose sensor signal.

In Example 9, the subject matter of Examples 1-8 includes, a carbon dioxide sensor connected to the perfusate circuit, the carbon dioxide sensor configured to produce a carbon dioxide signal based on a carbon dioxide level of the perfusate; and an oxygen sensor connected to the perfusate circuit, the oxygen sensor configured to produce an oxygen signal based on an oxygen level of the perfusate.

In Example 10, the subject matter of Example 9 includes, a controller configured to: receive the carbon dioxide signal and the oxygen signal; and operate the pump and the gas transfer unit based on the carbon dioxide signal and the oxygen signal.

In Example 11, the subject matter of Examples 1-10 includes, a sampling port connected to the perfusate circuit and configured to receive a syringe to withdraw a sample without compromising sterility of the perfusate within the circuit.

In Example 12, the subject matter of Examples 1-11 includes, a dosing port connected to the perfusate circuit and configured to deliver supplements to the perfusate.

In Example 13, the subject matter of Examples 1-12 includes, a pressure sensor connected to the perfusate circuit, the pressure sensor configured to produce a pressure signal based on a pressure of the perfusate within the perfusate circuit; and a controller configured to receive the pressure signal and operate the pump based on the pressure signal.

Example 14 is a system for testing and transporting a biologically engineered organ, the system comprising: a housing configured to receive and support a biologically engineered organ therein in a perfusate flow, the housing including a perfusate inlet and a perfusate outlet to receive the perfusate flow through the housing; a transportable enclosure surrounding at least a portion of the housing, the enclosure configured to insulate the housing; a perfusate circuit connected to the perfusate inlet and the perfusate outlet and configured to transmit perfusate through the system; a pump connected to the circuit and configured to circulate perfusate through the perfusate circuit; a gas transfer unit connected to the perfusate circuit and configured to transfer gas to and from the perfusate; a humidity sensor connected to the housing, the humidity sensor configured to produce humidity signal based on a humidity of the housing; a heating and cooling system connected to the perfusate circuit to exchange heat with the perfusate such that the perfusate is delivered to the housing within a target temperature range; and a controller configured to operate the pump, the gas transfer unit, and the heating and cooling system based on the humidity signal.

In Example 15, the subject matter of Example 14 includes, an inlet temperature sensor connected to the circuit upstream of the housing, the inlet temperature sensor configured to produce an inlet temperature signal based on an inlet temperature of the perfusate entering the housing; and an outlet temperature sensor connected to the circuit downstream of the housing, the outlet temperature sensor configured to produce an outlet temperature signal based on an outlet temperature of the perfusate leaving the housing.

In Example 16, the subject matter of Example 15 includes, wherein the controller is further configured to: operate the pump, the gas transfer unit, and the heating and cooling system based on the inlet temperature signal and the outlet temperature signal.

In Example 17, the subject matter of Examples 14-16 includes, a carbon dioxide sensor connected to the perfusate circuit, the carbon dioxide sensor configured to produce a carbon dioxide signal based on a carbon dioxide level of the perfusate; and an oxygen sensor connected to the perfusate circuit, the oxygen sensor configured to produce an oxygen signal based on an oxygen level of the perfusate.

In Example 18, the subject matter of Example 17 includes, wherein the controller is further configured to: receive the carbon dioxide signal and the oxygen signal; and operate the pump and the gas transfer unit based on the carbon dioxide signal and the oxygen signal.

In Example 19, the subject matter of Examples 14-18 includes, a pressure sensor connected to the perfusate circuit, the pressure sensor configured to produce a pressure signal based on a pressure of the perfusate within the perfusate circuit; wherein the controller is further configured to receive the pressure signal and operate the pump based on the pressure signal.

In Example 20, the subject matter of Examples 14-19 includes, an ammonia sensor connected to the perfusate circuit, the ammonia sensor configured to produce an ammonia signal based on an ammonia concentration of the perfusate within the perfusate circuit.

In Example 21, the subject matter of Example 20 includes, wherein the controller is further configured to receive the ammonia signal and operate the pump and the gas transfer unit based on the ammonia signal.

In Example 22, the subject matter of Examples 20-21 includes, wherein the controller is further configured to transmit an alert based on the ammonia sensor.

In Example 23, the subject matter of Examples 14-22 includes, a glutamine sensor connected to the perfusate circuit, the glutamine sensor configured to produce a glutamine signal based on a glutamine level of the perfusate within the perfusate circuit.

In Example 24, the subject matter of Example 23 includes, wherein the controller is further configured to receive the glutamine signal and operate the pump and the gas transfer unit based on the glutamine signal.

In Example 25, the subject matter of Examples 23-24 includes, wherein the controller is further configured to transmit an alert based on the glutamine signal.

In Example 26, the subject matter of Examples 14-25 includes, a lactate sensor connected to the perfusate circuit, the lactate sensor configured to produce a lactate signal based on a lactate level of the perfusate within the perfusate circuit.

In Example 27, the subject matter of Example 26 includes, wherein the controller is further configured to receive the lactate signal and operate the pump and the gas transfer unit based on the lactate signal.

In Example 28, the subject matter of Examples 26-27 includes, wherein the controller is further configured to transmit an alert based on the lactate signal.

In Example 29, the subject matter of Examples 14-28 includes, a pH sensor connected to the perfusate circuit, the pH sensor configured to produce a pH signal based on a pH level of the perfusate within the perfusate circuit.

In Example 30, the subject matter of Example 29 includes, wherein the controller is further configured to receive the pH signal and operate the pump and the gas transfer unit based on the pH signal.

In Example 31, the subject matter of Examples 29-30 includes, wherein the controller is further configured to transmit an alert based on the pH signal.

In Example 32, the subject matter of Examples 14-31 includes, a bile sensor connected to the perfusate circuit, the bile sensor configured to produce a bile signal based on a bile level of the perfusate within the perfusate circuit.

In Example 33, the subject matter of Example 32 includes, wherein the controller is further configured to receive the bile signal and operate the pump and the gas transfer unit based on the bile signal.

In Example 34, the subject matter of Examples 32-33 includes, wherein the controller is further configured to transmit an alert based on the bile signal.

In Example 35, the subject matter of Examples 14-34 includes, a creatine or albumin sensor connected to the perfusate circuit, the creatine or albumin sensor configured to produce a kidney signal based on a creatine or albumin level of the perfusate within the perfusate circuit.

In Example 36, the subject matter of Example 35 includes, wherein the controller is further configured to receive the kidney signal and operate the pump and the gas transfer unit based on the kidney signal.

In Example 37, the subject matter of Examples 35-36 includes, wherein the controller is further configured to transmit an alert based on the kidney signal.

Example 38 is a system for testing and transporting a biologically engineered organ, the system comprising: a housing configured to receive and support a biologically engineered organ therein in a perfusate flow, the housing including a perfusate inlet and a perfusate outlet to receive the perfusate flow through the housing; a transportable enclosure surrounding at least a portion of the housing, the enclosure configured to insulate the housing; a perfusate circuit connected to the perfusate inlet and the perfusate outlet and configured to transmit perfusate through the system; a pump connected to the circuit and configured to circulate perfusate through the perfusate circuit; a gas transfer unit connected to the perfusate circuit and configured to transfer gas to and from the perfusate; an organ health sensor connected to the circuit downstream of the housing, the glucose sensor configured to produce an organ health signal based on a health indicator in the perfusate; and a controller configured to operate the pump and the gas transfer unit based on the health indicator signal.

In Example 39, the subject matter of Example 38 includes, wherein the organ health sensor is one of an ammonia, glutamine, lactate, pH, bile, creatinine, albumin, oxygen, or carbon dioxide sensor.

In Example 40, the subject matter of Examples 38-39 includes, wherein the controller is further configured to receive the kidney signal and operate the pump and the gas transfer unit based on the kidney signal.

In Example 41, the subject matter of Examples 38-40 includes, wherein the controller is further configured to transmit an alert based on the kidney signal.

In Example 42, the subject matter of Examples 38-41 includes, wherein the controller is further configured to produce release criteria for the biologically engineered organ; and transmit the release criteria to a system located at a point of use of the biologically engineered organ.

Example 43 is a method for testing and maintaining health of a biologically engineered organ during transportation of the biologically engineered organ, the method comprising: receiving and supporting, in a housing, a biologically engineered organ; pumping, using a pump, perfusate through a perfusate circuit connected to the housing to flow perfusate through the housing; and transferring gas to and from the perfusate using the gas transfer unit connected to the perfusate circuit.

In Example 44, the subject matter of Example 43 includes, cooling the perfusate using a cooling system connected to the perfusate circuit.

In Example 45, the subject matter of Examples 43-44 includes, receiving a carbon dioxide signal and an oxygen signal from a carbon dioxide and oxygen sensor connected to the perfusate circuit; and modifying operation of the pump and the gas transfer unit based on the carbon dioxide signal and the oxygen signal.

Example 46 is at least one machine-readable medium including instructions that, when executed by processing circuitry, cause the processing circuitry to perform operations to implement of any of Examples 1-45.

Example 47 is an apparatus comprising means to implement of any of Examples 1-45.

Example 48 is a system to implement of any of Examples 1-45.

Example 49 is a method to implement of any of Examples 1-45.

Example 50 is any of the systems or devices discussed above including a storage tank or an expansion tank.

Example 51 is any of the systems or devices discussed above including one or more of an inlet system or an outlet system as described herein.

In Example 52, the apparatuses or method of any one or any combination of Examples 1 - 51 can optionally be configured such that all elements or options recited are available to use or select from.

The above detailed description includes references to the accompanying drawings, which form a part of the detailed description. The drawings show, by way of illustration, specific embodiments in which the invention can be practiced. These embodiments are also referred to herein as "examples." Such examples can include elements in addition to those shown or described. However, the present inventors also contemplate examples in which only those elements shown or described are provided. Moreover, the present inventors also contemplate examples using any combination or permutation of those elements shown or described (or one or more aspects thereof), either with respect to a particular example (or one or more aspects thereof), or with respect to other examples (or one or more aspects thereof) shown or described herein.

In the event of inconsistent usages between this document and any documents so incorporated by reference, the usage in this document controls. In this document, the terms "including" and "in which" are used as the plain-English equivalents of the respective terms "comprising" and "wherein." Also, in the following claims, the terms "including" and "comprising" are open-ended, that is, a system, device, article, composition, formulation, or process that includes elements in addition to those listed after such a term in a claim are still deemed to fall within the scope of that claim.

The above description is intended to be illustrative, and not restrictive. For example, the above-described examples (or one or more aspects thereof) can be used in combination with each other. Other embodiments can be used, such as by one of ordinary skill in the art upon reviewing the above description. The Abstract is provided to comply with 37 C.F.R. §1.72(b), to allow the reader to quickly ascertain the nature of the technical disclosure. It is submitted with the understanding that it will not be used to interpret or limit the scope or meaning of the claims. Also, in the above Detailed Description, various features can be grouped together to streamline the disclosure. This should not be interpreted as intending that an unclaimed disclosed feature is essential to any claim. Rather, inventive subject matter can lie in less than all features of a particular disclosed embodiment. Thus, the following claims are hereby incorporated into the Detailed Description as examples or embodiments, with each claim standing on its own as a separate embodiment, and it is contemplated that such embodiments can be combined with each other in various combinations or permutations. The scope of the invention should be determined with reference to the appended claims, along with the full scope of equivalents to which such claims are entitled.

The following is a non-limiting list of embodiments of the present invention:
Embodiment 1. A system for testing and transporting a biologically engineered organ, the system comprising:
   a housing configured to receive and support a biologically engineered organ therein in a perfusate flow, the housing including a perfusate inlet and a perfusate outlet to receive the perfusate flow through the housing;
   a transportable enclosure surrounding at least a portion of the housing, the enclosure configured to insulate the housing;
   a perfusate circuit connected to the perfusate inlet and the perfusate outlet and configured to transmit perfusate through the system;
   a pump connected to the circuit and configured to circulate perfusate through the perfusate circuit;
   a gas transfer unit connected to the perfusate circuit and configured to transfer gas to and from the perfusate;
   a glucose sensor connected to the circuit downstream of the housing, the glucose sensor configured to produce a glucose sensor signal based on a glucose level of the perfusate; and
   a controller configured to operate the pump and the gas transfer unit based on the glucose signal.
Embodiment 2. The system of Embodiment 1, further comprising: a heating and cooling system connected to the perfusate circuit to exchange heat with the perfusate such that the perfusate is delivered to the housing within a target temperature range.
Embodiment 3. The system of Embodiment 2, further comprising:
   an inlet temperature sensor connected to the circuit upstream of the housing, the inlet temperature sensor configured to produce an inlet temperature signal based on an inlet temperature of the perfusate entering the housing; and
   an outlet temperature sensor connected to the circuit downstream of the housing, the outlet temperature sensor configured to produce an outlet temperature signal based on an outlet temperature of the perfusate leaving the housing.
Embodiment 4. The system of Embodiment 3, further comprising a controller configured to: receive the inlet temperature signal and the outlet temperature signal; and operate the pump, the gas transfer unit, and the heating and cooling system based on the inlet temperature signal and the outlet temperature signal.
Embodiment 5. The system of Embodiment 2, further comprising: a humidity sensor connected to the housing, the humidity sensor configured to produce a humidity signal based on a humidity of the housing; and a controller configured to receive the humidity signal and operate the pump, the gas transfer unit, and the heating and cooling system based on the humidity signal.
Embodiment 6. The system of any of Embodiments1-5, further comprising: a power source supported by the enclosure and configured to power the pump and the gas transfer unit.
Embodiment 7. The system of any of Embodiments 1-6, further comprising: a metabolite sensor connected to the circuit downstream of the housing, the metabolite sensor configured to produce a metabolite sensor signal based on a metabolite level of the perfusate.
Embodiment 8. The system of Embodiment 4, further comprising a controller configured to: receive the metabolite sensor signal and the glucose sensor signal; and operate the pump and the gas transfer unit based on the metabolite sensor signal and the glucose sensor signal.
Embodiment 9. The system of any of Embodiments 1-8, further comprising: a carbon dioxide sensor connected to the perfusate circuit, the carbon dioxide sensor configured to produce a carbon dioxide signal based on a carbon dioxide level of the perfusate; and an oxygen sensor connected to the perfusate circuit, the oxygen sensor configured to produce an oxygen signal based on an oxygen level of the perfusate.
Embodiment 10. The system of Embodiment 9, further comprising a controller configured to: receive the carbon dioxide signal and the oxygen signal; and operate the pump and the gas transfer unit based on the carbon dioxide signal and the oxygen signal.
Embodiment 11. The system of any of Embodiments 1-10, further comprising: a sampling port connected to the perfusate circuit and configured to receive a syringe to withdraw a sample without compromising sterility of the perfusate within the circuit.
Embodiment 12. The system of any of Embodiments 1-11, further comprising: a dosing port connected to the perfusate circuit and configured to deliver supplements to the perfusate.
Embodiment 13. The system of any of Embodiments 1-12, further comprising: a pressure sensor connected to the perfusate circuit, the pressure sensor configured to produce a pressure signal based on a pressure of the perfusate within the perfusate circuit; and a controller configured to receive the pressure signal and operate the pump based on the pressure signal.
Embodiment 14. A method for testing and maintaining health of a biologically engineered organ during transportation of the biologically engineered organ, the method comprising: receiving and supporting, in a housing, a biologically engineered organ; pumping, using a pump, perfusate through a perfusate circuit connected to the housing to flow perfusate through the housing; and transferring gas to and from the perfusate using the gas transfer unit connected to the perfusate circuit.
Embodiment 15. The method of Embodiment 14, further comprising: cooling the perfusate using a cooling system connected to the perfusate circuit; receiving a carbon dioxide signal and an oxygen signal from a carbon dioxide and oxygen sensor connected to the perfusate circuit; and modifying operation of the pump and the gas transfer unit based on the carbon dioxide signal and the oxygen signal.

## Claims

1. A system (200) for providing external organ function to a patient in need, the system (200) comprising:
a biologically engineered organ (50);
a housing (204) configured to support the biologically engineered organ (50) therein in a perfusate flow, the housing including a perfusate inlet (204a) and a perfusate outlet (204b);
a perfusate circuit (206) connected to the perfusate inlet (204a) and the perfusate outlet (204b) and configured to transmit perfusate through the system (200); and
a pump (208) connected to the perfusate circuit (206) and configured to circulate the perfusate through the perfusate circuit (206).

2. The system of claim 1, further comprising a controller (216) configured to operate the pump to circulate perfusate through the perfusate circuit, wherein the perfusate comprises the patient's blood.

3. The system of claim 2, wherein the perfusate inlet (204a) and perfusate outlet (204b) are connected directly to the patient's blood through patient access lines that extend beyond the housing and are configured to receive the perfusate flow through the perfusate circuit (206).

4. The system of claim 3, wherein the pump is configured to circulate the patient's blood received from the patient access lines through the perfusate circuit, such that the perfusate includes the patient's.

5. The system of claim 1, further comprising a transportable enclosure (202) surrounding at least a portion of the housing (204), the enclosure (202) configured to insulate the housing (204).

6. The system of claim 2, further comprising a gas transfer unit (212) connected to the perfusate circuit (206) and configured to transfer gas to and from the perfusate.

7. The system of claim 3, further comprising a glucose sensor (224) connected to the perfusate circuit (206) downstream of the housing (204), the glucose sensor (224) configured to produce a glucose sensor signal based on a glucose level of the perfusate or the patient's blood.

8. The system of claim 2, further comprising a heating and cooling system (210) connected to the perfusate circuit to exchange heat with the perfusate such that the perfusate is delivered to the housing within a target temperature range.

9. The system of claim 2, further comprising:
an inlet temperature sensor (218) connected to the circuit upstream of the housing, the inlet temperature sensor configured to produce an inlet temperature signal based on an inlet temperature of the perfusate entering the housing; and
an outlet temperature sensor (220) connected to the circuit downstream of the housing, the outlet temperature sensor configured to produce an outlet temperature signal based on an outlet temperature of the perfusate leaving the housing.

10. The system of claim 9, wherein the controller is configured to:
receive the inlet temperature signal and the outlet temperature signal; and
operate the pump, the gas transfer unit, and the heating and cooling system based on the inlet temperature signal and the outlet temperature signal.

11. The system of claim 2, further comprising:
a humidity sensor (222) connected to the housing, the humidity sensor configured to produce a humidity signal based on a humidity of the housing; and
the controller being configured to receive the humidity signal and operate the pump, the gas transfer unit, and the heating and cooling system based on the humidity signal.

12. The system of claim 2, further comprising a metabolite sensor (226) connected to the circuit downstream of the housing, the metabolite sensor configured to produce a metabolite sensor signal based on a metabolite level of the perfusate.

13. The system of claim 12, wherein the controller is configured to:
receive the metabolite sensor signal and the glucose sensor signal; and
operate the pump and the gas transfer unit based on the metabolite sensor signal and the glucose sensor signal.

14. The system of claim 2, further comprising:
a carbon dioxide sensor (228) connected to the perfusate circuit, the carbon dioxide sensor configured to produce a carbon dioxide signal based on a carbon dioxide level of the perfusate; and
an oxygen sensor (228) connected to the perfusate circuit, the oxygen sensor configured to produce an oxygen signal based on an oxygen level of the perfusate.

15. The system of claim 14, wherein the controller is configured to:
receive the carbon dioxide signal and the oxygen signal; and
operate the pump and the gas transfer unit based on the carbon dioxide signal and the oxygen signal.

16. The system of claim 1, further comprising:
a dosing port (236) connected to the perfusate circuit and configured to deliver supplements to the perfusate.

17. The system of claim 2, further comprising:
a pressure sensor (232) connected to the perfusate circuit, the pressure sensor configured to produce a pressure signal based on a pressure of the perfusate within the perfusate circuit; and
the controller being configured to receive the pressure signal and operate the pump based on the pressure signal.

18. The system of claim 1, wherein the biologically engineered organ is a biologically engineered liver or lung.

19. The system of claim 1, wherein the biologically engineered organ is a liver and the external organ function comprises a reduction of ammonia levels in the perfusate or blood of the patient.

20. A method providing external organ function to a patient in need, the method comprising:
supporting, in a housing comprising a perfusate inlet and a perfusate outlet, a biologically engineered organ, wherein the perfusate inlet and
perfusate outlet are connected to a perfusate circuit that circulates perfusate through the biologically engineered organ;
connecting the biologically engineered organ to the patient.

21. The method of claim 20, wherein the biologically engineered organ is a biologically engineered liver or lung, optionally, wherein the organ is connected to the patient through one or more patient access lines connected directly to the patient's blood, and optionally, circulating the patient's blood through the perfusate circuit as perfusate, such that the patient's blood is circulated through the vasculature of the biologically engineered organ and back to the patient in order to provide external organ support to the patient.

22. The method of claim 20, wherein the biologically engineered organ is a
liver and the external organ function comprises a reduction of ammonia levels in the perfusate or blood.
